# EUROPÄISCHE PATENTANMELDUNG

(11) **EP 1 262 167 A1**
(43) Veröffentlichungstag der Anmeldung: **04.12.2002**
(21) Anmeldenummer: 01401428.6
(22) Anmeldetag: 01.06.2001
(51) Int. Cl.: A61K 7/48, A61K 7/06

(54) **Kosmetische Zubereitungen enthaltend ein Extrakt von keimenden Pflanzen**

(71) Anmelder: Cognis France S.A., 31360 Saint-Martory (FR)
(72) Erfinder: Moussou, Philippe, 54000 Nancy (FR); Danoux, Louis, 54420 Saulxures-Les-Nancy (FR); Daridon, Bruno, 54220 Malzeville (FR); Pauly, Gilles, 54000 Nancy (FR)
(74) Vertreter: Cabinet HERRBURGER

(57) **Zusammenfassung**

Vorgeschlagen werden neue kosmetische Zubereitungen, welche eine wirksame Menge eines Extraktes von keimenden Pflanzen enthalten.

## Beschreibung

### Gebiet der Erfindung

Die Erfindung befindet sich auf dem Gebiet der Kosmetik und betrifft neue Zubereitungen mit einem wirksamen Gehalt an Extrakten keimender Pflanzen.

### Stand der Technik

An moderne kosmetische Zubereitungen werden von der Verbraucherin immer höhere Anforderungen gestellt. Längst ist die Zeit vorbei, in der es ausgereicht hat, wenn eine Nachtcreme der Haut Feuchtigkeit zurückgegeben hat, heute soll die Zubereitung gleichzeitig auch vor Umweltstress schützen und Schädigungen reparieren. Abgesehen davon, dass solche Anforderungen ein tiefes Verständnis der biochemischen Vorgänge in Haut und Haaren erfordert, welches auch heute noch nicht vollständig vorhanden ist, werden für diese Forderung in der Regel sehr viele unterschiedliche Wirkstoffe benötigt, die der Vielzahl von auslösenden Faktoren Rechnung tragen. Abgesehen von der möglicherweise unerwünschten Interaktion solcher künstlich zusammengestellten Stoffgemische besteht natürlich auch das Problem der Lagerhaltung und des technischen Aufwandes bei der Herstellung, das solche komplexen Produkte schwierig in der Entwicklung und anspruchsvoll im Preis machen. Andererseits ist sofort verständlich, dass seitens der Kosmetikindustrie ein großes Interesse an solchen zumal pflanzlichen Wirkstoffen besteht, welche über eine "Breitbandwirkung" verfügen, d.h. unterschiedliche kosmetische Problemstellungen gleichzeitig lösen. Dabei sind natürlich solche natürlichen Prozesse in Pflanzen von besonderem Interesse, in deren Verlauf eine Vielzahl von Stoffen nebeneinander entstehen und sich in ihrer Aktivität ergänzen.

Unter dem Begriff Keimung werden ganz verschiedene biochemische Abläufe zusammengefasst, wie z.B. die Proteinhydration, subzellulare Strukturänderungen, Atmung, Synthese von Makromolekülen sowie die generelle Ausdehnung der Zellen. Gemeinsames Ziel all dieser Prozesse ist es, den in der ruhenden Saat enthaltenen dehydrierten Pflanzenembryo in eine keimende Sprosse zu verwandeln. Zu diesem Zweck sind bestimmte Umweltbedingungen, beispielsweise eine geeignete Temperatur und die Gegenwart von Sauerstoff erforderlich. Während des Vorgangs der Keimung sind eine Vielzahl von Stoffen involviert, insbesondere Wachstumsfaktoren, wie z.B. Auxine, Gibberelline und Cytokine sowie Enzyme, die die Mobilisierung von Speicherstoffen, wie etwa Kohlenhydraten, Proteinen, Triacylglycerin oder Phytin bewirken. Der Organismus selbst produziert endogene Enzyme (Amylasen, Pentosanasen, Glucanasen, Proteinasen, Lipasen, Phytasen etc.) um die Makromoleküle zu metabolisieren, damit die kleineren Bruchstücke an den Ort transportiert werden können, an dem sie benötigt werden.

In diesem Zusammenhang sei beispielsweise auf einen Aufsatz von Malak [**Cosmetol. 20, 44 (1998)**] hingewiesen, in dem die Verwendung eines Malzextraktes als Inhibitor für Matrix Metalloproteinasen, wie beispielsweise Collagenase beschrieben wird. Tronnier berichtet in **Ärztl. Kosmetol. 17, 342-352 (1987)** auch über dessen anti-inflammatorische Wirkung. Aus der DE 4141866 A1 ist die immunostimulierende Wirkung von Extrakten keimender Pflanzen bekannt. Von Benaiges et al. wird in **NCP, 224, 4-7 (1997)** über die Anti-Elastase-Wirkung von Extrakten keimender Alfalfa-Sprossen berichtet. Gegenstand der **WO 99/56712** (Provital) ist der Einsatz von Extrakten keimender Pflanzen zur Stimulation der Zellatmung. In der **FR 2665900 A1** (Andromaco) wird der Einsatz von Oligosacchariden, die aus keimenden Pflanzen gewonnen werden, zur Wundbekämpfung beschrieben. Offenbart wird dabei die Stimulation der Lymphoblasten, bei denen es sich jedoch weder um Haut- noch um Haarzellen handelt. Schließlich beschreibt die **FR 2747044 A1** (Coletica) die Verwendung von Superoxid Dismutase, die aus keimenden pflanzen gewonnen wird, in kosmetischen Zubereitungen.

Die Aufgabe der Erfindung hat folglich darin bestanden, neue kosmetische Wirkstoffe unter Einsatz pflanzlicher Wirkstoffe zur Verfügung zu stellen, die unterschiedlichste Probleme gleichzeitig bekämpfen. Insbesondere sollten die Stoffe der Hautalterung und der Erschlaffung des Bindegewebes (Cellulits) entgegenwirken, die Lipidsynthese im Stratum Corneum stimulieren und so die Hautbarriere stärken, Haut- und Haarfollikeln beim Schutz gegen Umweltgifte, oxidativen Stress und UV-Strahlung unterstützen, die Synthese von Makromolekülen, wie z.B. Kollagen in den Fibroblasten fördern, die Inflammation von empfindlicher menschlicher Haut verhindern sowie die Lypolyse und die Entschlackung von Körperzellen unterstützen.

### Beschreibung der Erfindung

Gegenstand der Erfindung sind kosmetische Zubereitungen, enthaltend eine wirksame Menge eines Extraktes von keimenden Pflanzen.

Überraschenderweise wurde gefunden, dass die Extrakte über das gewünschte breite Spektrum von Wirkungen verfügen, und daher für die Pflege und den Schutz von Haut und Haaren eingesetzt werden können.

### Pflanzenextrakte

Keimende Pflanzen enthalten innerhalb gewisser Grenzen stets die gleichen Wirkstoffe, mit deren Hilfe im Sinne der Erfindung die beschriebene breite kosmetische Wirkung erzielt werden kann. Ihre Auswahl ist daher wenig kritisch. Typische Beispiele sind aber Alfalfa, Bambaranuss, Bockshorn, Borretsch, Broccoli, Buchweizen, Chinakohl, Erbse, Erdnuss, Flachs, Fenchel, Gerste, Getreide, Gewürznelke, Hafer, Karotte, Kresse, Linsen, Mais, Melone, Petersilie, Raps, Rettich, Reis, Roggen, Rotkohl, Sellerie, Senf, Sesam, Soja, Sonnenblume, Weizen, und Zwiebel. Selbstverständlich können auch Mischungen eingesetzt werden. Die wirksame Menge der Extrakte liegt in der Regel bei 0,01 bis 5, vorzugsweise 0,1 bis 2 und insbesondere 0,05 bis 1 Gew.-% - wobei sich diese Angaben selbstverständlich auf den Aktivstoffgehalt in den Extrakten und den Anteil der Extrakte an der Endformulierung beziehen.

### Extraktion

Die Herstellung der Extrakte kann in an sich bekannter Weise erfolgen, d.h. beispielsweise durch wässrigen, alkoholischen oder wässrigalkoholischen Auszug der Pflanzen bzw. Pflanzenteile. Bezüglich der geeigneten herkömmlichen Extraktionsverfahren wie der Mazeration, der Remazeration, der Digestion, der Bewegungsmazeration, der Wirbelextraktion, Ultraschallextraktion, der Gegenstromextraktion, der Perkolation, der Reperkolation, der Evakolation (Extraktion unter vermindertem Druck), der Diakolation und Festflüssig-Extraktion unter kontinuierlichem Rückfluss die in einem Soxhlet-Extraktor durchgeführt wird, die dem Fachmann geläufig und im Prinzip alle anwendbar sind, sei der Einfachheit halber beispielsweise auf **Hagers Handbuch der Pharmazeutischen Praxis,** (5. Auflage, Bd. 2, S. 1026-1030, Springer Verlag, Berlin-Heidelberg-New-York 1991) verwiesen. Für den großtechnischen Einsatz vorteilhaft ist die Perkolationsmethode. Als Ausgangsmaterial können frische Pflanzen oder Pflanzenteile eingesetzt werden, üblicherweise wird jedoch von getrockneten Pflanzen und/oder Pflanzenteilen ausgegangen, die vor der Extraktion mechanisch zerkleinert werden können. Hierbei eignen sich alle dem Fachmann bekannten Zerkleinerungsmethoden, als Beispiel sei die Gefriermahlung genannt. Als Lösungsmittel für die Durchführung der Extraktionen können organische Lösungsmittel, Wasser (vorzugsweise heißes Wasser einer Temperatur von über 80 °C und insbesondere von über 95 °C) oder Gemische aus organischen Lösungsmitteln und Wasser, insbesondere niedermolekulare Alkohole mit mehr oder weniger hohen Wassergehalten, verwendet werden. Besonders bevorzugt ist die Extraktion mit Methanol, Ethanol, Pentan, Hexan, Heptan, Aceton, Propylenglykolen, Polyethylenglykolen sowie Ethylacetat sowie Mischungen hieraus sowie deren wässrige Gemische. Die Extraktion erfolgt in der Regel bei 20 bis 100 °C, bevorzugt bei 30 bis 90 °C, insbesondere bei 60 bis 80 °C. In einer bevorzugten Ausführungsform erfolgt die Extraktion unter Inertgasatmosphäre zur Vermeidung der Oxidation der Wirkstoffe des Extraktes. Dies ist insbesondere bei Extraktionen bei Temperaturen über 40 °C von Bedeutung. Die Extraktionszeiten werden vom Fachmann in Abhängigkeit vom Ausgangsmaterial, dem Extraktionsverfahren, der Extraktionstemperatur, vom Verhältnis Lösungsmittel zu Rohstoff u.a. eingestellt. Nach der Extraktion können die erhaltenen Rohextrakte gegebenenfalls weiteren üblichen Schritten, wie beispielsweise Aufreinigung, Konzentration und/oder Entfärbung unterzogen werden. Falls wünschenswert, können die so hergestellten Extrakte beispielsweise einer selektiven Abtrennung einzelner unerwünschter Inhaltsstoffe, unterzogen werden. Die Extraktion kann bis zu jedem beliebigen Extraktionsgrad erfolgen, wird aber gewöhnlich bis zur Erschöpfung durchgeführt. Typische Ausbeuten (= Trockensubstanzmenge des Extraktes bezogen auf eingesetzte Rohstoffmenge) bei der Extraktion getrockneter Blätter liegen im Bereich von 3 bis 15, insbesondere 6 bis 10 Gew.-%. Die vorliegenden Erfindung umfasst die Erkenntnis, dass die Extraktionsbedingungen sowie die Ausbeuten der Endextrakte vom Fachmann ja nach gewünschtem Einsatzgebiet gewählt werden können. Diese Extrakte, die in der Regel Aktivsubstanzgehalte (= Feststoffgehalte) im Bereich von 0,5 bis 10 Gew.-% aufweisen, können als solche eingesetzt werden, es ist jedoch ebenfalls möglich, das Lösungsmittel durch Trocknung, insbesondere durch Sprüh- oder Gefriertrocknung vollständig zu entfernen, wobei ein intensiv rot gefärbter Feststoff zurückbleibt. Die Extrakte können auch als Ausgangsstoffe für die Gewinnung der oben genannten reinen Wirkstoffe dienen, sofern diese nicht auf synthetischem Wege einfacher und kostengünstiger hergestellt werden können. Demzufolge kann der Wirkstoffgehalt in den Extrakten 5 bis 100, vorzugsweise 50 bis 95 Gew.-% betragen. Die Extrakte selbst können als wässrige und/oder in organischen Solventien gelöste Zubereitungen sowie als sprüh- bzw. gefriergetrocknete, wasserfreie Feststoffe vorliegen. Als organische Lösungsmittel kommen in diesem Zusammenhang beispielsweise die aliphatischen Alkohole mit 1 bis 6 Kohlenstoffatomen (z.B. Ethanol), Ketone (z.B. Aceton), Halogenkohlenwasserstoffe (z.B. Chloroform oder Methylenchlorid), niedere Ester oder Polyole (z.B. Glycerin oder Glycole) in Frage.

In einer bevorzugten Ausführungsform der Erfindung erfolgt die Herstellung der Extrakte jedoch dergestalt, dass man die keimenden Samen zunächst mit Wasser und/oder Alkohol extrahiert und dann in einem ersten Schritt thermisch behandelt, wobei die Temperatur langsam bis auf 100 °C angehoben wird, um die Enzyme, die zur Metabolisierung der Speicherstoffe erforderlich sind, zu aktivieren. Nach einer gewissen Zeit wird die Temperatur auf über 100 °C angehoben, um diese Enzyme wieder zu desaktivieren. Anschließend erfolgt - optional - der Zusatz von Exogenasen, die die von den in den Extrakten bereits enthaltenen Endogenasen begonnene Hydrolyse abschließen sollen. Anschließend kann wie oben beschrieben die Trocknung der Extrakte erfolgen.

### Gewerbliche Anwendbarkeit

Wie schon oben erläutert betrifft ein Schwerpunkt der Erfindung generell die Verwendung von Extrakten keimender Pflanzen zur Herstellung von kosmetischen Zubereitungen, in denen sie in Mengen von 0,01 bis 25, vorzugsweise 0,1 bis 15 und insbesondere 1 bis 5 Gew.-% enthalten sein können. Weitere Gegenstände der Erfindung betreffen die Verwendung der Extrakte
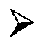 zur Stimulation des Zellwachstums und des Zellmetabolismus;
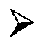 zur Stimulation der Erneuerung von dermalen Makromolekülen durch die Fibroblasten;
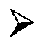 zur Stimulation der Zellproteinsynthese zum Schutz gegen spontane Alterungseffekte;
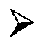 zur Steigerung der Protein- und GSH-Konzentration in den Zellen (Stärkung der Abwehrkräfte gegen Umwelteinflüsse);
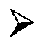 zur Stimulierung der G6PDH-Aktivität gegen trockene und raue Haut;
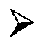 zur Immunmodulation;
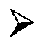 als anti-inflammatorische Wirkstoffe;
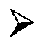 als Wirkstoffe gegen Akne und Rosacea;
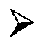 als Antioxidantien;
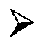 zum Schutz von Haut und Haaren gegen den Einfluss von UVA- und UVB-Strahlen;
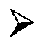 zum Schutz von empfindlicher Haut;
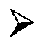 als Anti-Stress-Wirkstoffe;
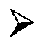 zur Stimulation der Synthese und Freisetzung von Hitzeschock-Proteinen;
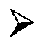 als lypolytische Wirkstoffe;
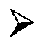 als Wirkstoffe zur Entschlackung von Körperzellen;
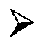 als Wirkstoffe zur Inhibierung der Melaninsynthese in Haut- und Haarzellen;
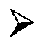 als Wirkstoffe mit östrogenartiger Aktivität.

### Kosmetische Zubereitungen

Die kosmetichen Zubereitungen, speziell die Hautbehandlungsmittel für empfindliche Haut, können als weitere Hilfs- und Zusatzstoffe milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

### Tenside

Als oberflächenaktive Stoffe können anionische, nichtionische, kationische und/oder amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124** oder **J.Falbe (ed.), "Katalysatoren, Tenside und** **Mineralöladditive", Thieme Verlag, Stuttgart, 1978, S. 123-217** verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen (vgl. **DE 19756377 A1**), insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Emulgatoren

Als Emulgatoren kommen beispielsweise nichtionogene Tenside aus mindestens einer der folgenden Gruppen in Frage:
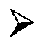 Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
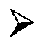 Alkyl- und/oder Alkenyloligoglykoside mit 8 bis 22 Kohlenstoffatomen im Alk(en)ylrest und deren ethoxylierte Analoga;
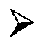 Anlagerungsprodukte von 1 bis 15 Mol Ethylenoxid an Ricinusöl und/oder gehärtetes Ricinusöl;
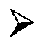 Anlagerungsprodukte von 15 bis 60 Mol Ethylenoxid an Ricinusöl und/oder gehärtetes Ricinusöl;
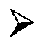 Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
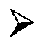 Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
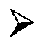 Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin.
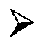 Mono-, Di- und Trialkylphosphate sowie Mono-, Di- und/oder Tri-PEG-alkylphosphate und deren Salze;
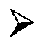 Wollwachsalkohole;
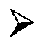 Polysiloxan-Polyalkyl-Polyether-Copolymere bzw. entsprechende Derivate;
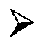 Block-Copolymere z.B. Polyethylenglycol-30 Dipolyhydroxystearate;
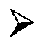 Polymeremulgatoren, z.B. Pemulen-Typen (TR-1,TR-2) von Goodrich;
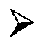 Polyalkylenglycole sowie
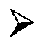 Glycerincarbonat.

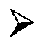 Ethylenoxidanlagerungsprodukte

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

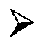 Alkyl- und/oder Alkenyloligoglykoside

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

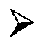 Partialglyceride

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendigtycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

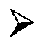 Sorbitanester

Als Sorbitanester kommen Sorbitanmonoisostearat, Sorbitansesquiisostearat, Sorbitan-diisostearat, Sorbitantriisostearat, Sorbitanmonooleat, Sorbitansesquioleat, Sorbitan-dioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesqui-tartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

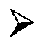 Polyglycerinester

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

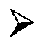 Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

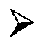 Amphotere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammonium-glycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropion-säuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### Fette und Wachse

Typische Beispiele für Fette sind Glyceride, d.h. feste oder flüssige pflanzliche oder tierische Produkte, die im wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacylsn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z.B. Magnesium-, Aluminium- und/oder Zinkstearat bzw. -ricinoleat eingesetzt werden.

### Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide, wie z.B. beschrieben in der **FR 2252840 A** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ AcrylatCopolymere, Octylacrylamid/Methylmeth-acrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage. Weitere geeignete Polymere und Verdickungsmittel sind in **Cosm.Toil. 108, 95 (1993)** aufgeführt.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosidund/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil. 91, 27 (1976).**

### UV-Lichtschutzfilter und Antioxidantien

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
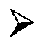 3-Benzylidencampher bzw. 3-Benzylidennorcampher und dessen Derivate, z.B. 3-(4-Methylbenzyliden)campher wie in der **EP 0693471 B1** beschrieben;
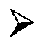 4-Aminobenzoesäurederivate, vorzugsweise 4-(Dimethylamino)benzoesäure-2-ethyl-hexylester, 4-(Dimemylamino)benzoesäure-2-octylester und 4-(Dimethylamino)benzoe-säureamylester;
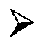 Ester der Zimtsäure, vorzugsweise 4-Methoxyzimtsäure-2-ethylhexylester, 4-Methoxy-zimtsäurepropylester, 4-Methoxyzimtsäureisoamylester 2-Cyano-3,3-phenylzimtsäure-2-ethylhexylester (Octocrylene);
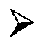 Ester der Salicylsäure, vorzugsweise Salicylsäure-2-ethylhexylester, Salicylsäure-4-iso-propylbenzylester, Salicylsäurehomomenthylester;
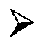 Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
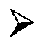 Ester der Benzalmalonsäure, vorzugsweise 4-Methoxybenzmalonsäuredi-2-ethylhexyl-ester;
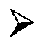 Triazinderivate, wie z.B. 2,4,6-Trianilino-(p-carbo-2'-ethyl-1'hexyloxy)-1,3,5-triazin und Octyl Triazon, wie in der **EP 0818450 A1** beschrieben oder Dioctyl Butamido Triazone (Uvasorb® HEB);
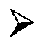 Propan-1,3-dione, wie z.B. 1-(4-tert.Butylphenyl)-3-(4'methoxyphenyl)propan-1,3-dion;
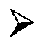 Ketotricyclo(5.2.1.0)decan-Derivate, wie in der **EP 0694521 B1** beschrieben.

### Als wasserlösliche Substanzen kommen in Frage:

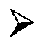 2-Phenylbenzimidazol-5-sulfonsäure und deren Alkali-, Erdalkali-, Ammonium-, Alkylammonium-, Alkanolammonium- und Glucammoniumsalze;
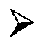 Sulfonsäurederivate von Benzophenonen, vorzugsweise 2-Hydroxy-4-methoxybenzo-phenon-5-sulfonsäure und ihre Salze;
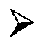 Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der **DE 19712033 A1** (BASF). Die UV-A und UV-B-Filter können selbstverständlich auch in Mischungen eingesetzt werden. Besonders günstige Kombinationen bestehen aus den Derivate des Benzoylmethans" z.B. 4-tert.-Butyl-4'methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in **SÖFW-Journal 122, 543 (1996)** sowie **Parf.Kosm. 3, 11 (1999)** zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

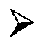 Keimhemmende Mittel

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

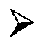 Enzyminhibitoren

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

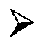 Geruchsabsorber

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

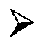 Antitranspirantien

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
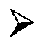 adstringierende Wirkstoffe,
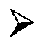 Ölkomponenten,
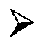 nichtionische Emulgatoren,
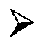 Coemulgatoren,
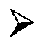 Konsistenzgeber,
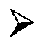 Hilfsstoffe wie z. B. Verdicker oder Komplexierungsmittel und/oder
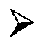 nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirko-nium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
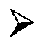 entzündungshemmende, hautschützende oder wohlriechende ätherische Öle,
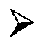 synthetische hautschützende Wirkstoffe und/oder
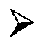 öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Quellmittel

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

### Insekten-Repellentien

Als Insekten-Repellentien kommen N,N-Diethyl-m-toluamid, 1,2-Pentandiol oder Ethyl Butylacetylaminopropionate in Frage

### Selbstbräuner und Depigmentierungsmittel

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
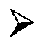 Glycerin;
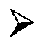 Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
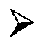 technische Oligoglyceringemische mit einem Eigenkondensationsgrad von 1,5 bis 10 wie etwa technische Diglyceringemische mit einem Diglyceringehalt von 40 bis 50 Gew.-%;
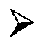 Methyolverbindungen, wie insbesondere Trimethylolethan, Trimethylolpropan, Trimethylolbutan, Pentaerythrit und Dipentaerythrit;
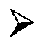 Niedrigalkylglucoside, insbesondere solche mit 1 bis 8 Kohlenstoffen im Alkylrest, wie beispielsweise Methyl- und Butylglucosid;
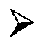 Zuckeralkohole mit 5 bis 12 Kohlenstoffatomen, wie beispielsweise Sorbit oder Mannit,
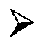 Zucker mit 5 bis 12 Kohlenstoffatomen, wie beispielsweise Glucose oder Saccharose;
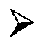 Aminozucker, wie beispielsweise Glucamin;
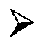 Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt. Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### Farbstoffe

Als Farbstoffe können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Beispiele

**Keimung.** Die Pflanzensaaten wurden über einen Zeitraum von 5 bis 14 h bei 14 °C in Wasser eingeweicht (alternativ: abwechselnd in Wasser eingeweicht und an der Luft getrocknet) und dann in speziellen rotierenden und belüfteten Kulturschalen über einen Zeitraum von 31 bis 233 h bei 25 °C zum Keimen gebracht.

**Herstellbeispiel H1.** Linsen wurde innerhalb von 7 Tagen zum Keimen gebracht und dann schockgefroren. 440 g der gefrorenen Keime wurden gemahlen und in 660 ml Wasser suspendiert. Die Suspension wurde 90 min bei 20 °C gerührt und die unlöslichen Bestandteile durch Zentrifugation und Filtration entfernt. Anschließend wurde das Filtrat entwässert und gefriergetrocknet.

**Herstellbeispiel H2.** 500 g der gefrorenen Linsenkeime wurden gemahlen und in 11 Wasser suspendiert. Die Suspension wurde 1 h bei 20 °C gerührt und die Temperatur dann innerhalb von 2 h schrittweise bis auf 90 °C angehoben. Anschließend wurde die festen Bestandteile wieder abgetrennt und die Extrakte nach Eindampfen lyophilisiert.

**Herstellbeispiel H3.** Sonnenblumenkörner wurde innerhalb von 7 Tagen zum Keimen gebracht und dann schockgefroren. 500 g der gefrorenen Keime wurden gemahlen und in 750 ml Wasser suspendiert. Die Suspension wurde 1 h bei 20 °C gerührt und dann für 15 min auf 100 °C erhitzt. Anschließend wurde die festen Bestandteile wieder abgetrennt und die Extrakte nach Eindampfen lyophilisiert.

**Herstellbeispiel H4.** Sonnenblumenkörner wurde innerhalb von 1 Tag zum Keimen gebracht und dann schockgefroren. 500 g der gefrorenen Keime wurden gemahlen und in 750 ml Wasser suspendiert. Die Suspension wurde 1 h bei 20 °C gerührt und dann für 15 min auf 100 °C erhitzt. Anschließend wurde die festen Bestandteile wieder abgetrennt und die Extrakte nach Eindampfen lyophilisiert.

**Herstellbeispiel H5.** Haferkörner wurde innerhalb von 26 Tagen zum Keimen gebracht und dann schockgefroren. 300 g der gefrorenen Keime wurden gemahlen und in 450 ml Wasser suspendiert. Die Suspension wurde 60 min bei 20 °C gerührt und die unlöslichen Bestandteile durch Zentrifugation und Filtration entfernt. Anschließend wurde das Filtrat entwässert und gefriergetrocknet.

**Herstellbeispiel H6.** 450 g der gefrorenen Haferkeime wurden gemahlen und in einer Mischung aus 2,5 1 Methanol und 1 1 Wasser suspendiert. Anschließend wurden die Keime über einen Zeitraum von 1 h unter Rückfluss extrahiert. Nach dem Abkühlen wurde das Methanol im Vakuum entfernt und der Extrakt lyophilisiert.

**Herstellbeispiel H7.** Roggenkörner wurden innerhalb von 31 Tagen zum Keimen gebracht und dann schockgefroren. 200 g der gefrorenen Keime wurden gemahlen und in 400 ml Wasser suspendiert. Die Suspension wurde 60 min bei 20 °C gerührt und die unlöslichen Bestandteile durch Zentrifugation und Filtration entfernt. Anschließend wurde das Filtrat entwässert und gefriergetrocknet.

### A. Wirksamkeit gegen Hautalterung

Das Enzym Glucose-6-phosphat Dehydrogenase (G6PDH) katalysiert den ersten Schritt des sogenannten "Pentose-Wegs", bei dem ein wesentlicher DNA-Baustein, nämlich die Desoxyribose gebildet wird. Dabei wird Glucose-6-phosphat (G6P) mit Hilfe von G6PDH in 6-Phosphatogluconat (6PG) überführt. Gleichzeitig wird das dabei erforderliche Coenzym NADP wird zu NADPH2 reduziert, welches seinerseits eine Vielzahl von anderen biologischen Reaktion wie z.B. das Recycling von Glutathion oder die Lipidsynthese katalysieren kann. Reduziertes Glutathion schützt viele Enzyme, die über SH-Gruppen verfügen und Zellen vor oxidativem Stress, wie beispielsweise UV-Einwirkung. Damit ist der G6PDH-Gehalt ein wichtiger Parameter für den Zellschutz und die die Hauterneuerung. Die G6PDH-Aktivität wurde in-vitro an menschlichen Fibroblasten nach der enzymatischen Methode von Okada bestimmt, der DNA-Gehalt nach dem Verfahren von Desaulniers. Die Ergebnisse sind in Tabelle 1 zusammengefasst. Angegeben sind die Ergebnisse von jeweils 3 Messreihen mit Dreifachbestimmung in %-rel gegenüber einer Blindprobe.

**Tabelle 1**

| **Stimulierung der G6PDH-Aktivität (Angaben in %-rel.)** | | | | | |
|---|---|---|---|---|---|
| **Extrakt** | **Konz. % w/v** | **DNA** | | **G6PDH** | |
| | | **nach 3 d** | **nach 6 d** | **nach 3 d** | **nach 6 d** |
| **Blindprobe** | **0** | **100** | **100** | **100** | **100** |
| H1 | 0,1 | 152 | 145 | 138 | 100 |
| H3 | 0,1 | 135 | 107 | 181 | 221 |
| H5 | 0,1 | 93 | 89 | 154 | 204 |
| H7 | 0,03 | 74 | 116 | 118 | 103 |

### B. Regenerative und wachstumsstimulierende Wirkung

Nach einer Inkubation von 72 h in einer Nährlösung bilden Fibroblasten gesättigte Monolayer aus, die Fibroblasten stellen ihre Aktivität ein und das Wachstum kommt zum Stillstand. Der Zellbrennstoff Adenosintriphosphat (ATP), der im wesentlichen in den Mitochondrien entsteht, wird zur Aktivierung von bestimmten Enzymen benötigt, die beispielsweise das Zellskelett kontrollieren, die Ionenkanäle, die Aufnahme von Nährstoffe und eine ganze Reihe anderer wichtiger biologischer Abläufe. Die Bestimmung des Proteingehaltes in den Zellen erfolgte nach der Methode von Bradford [vgl. Anal. Biochem. 72, 248-254 (1977) ]. Glutathion (GSH) ist ein spezielles Protein, welches von den Zellen zum Schutz gegen oxidativen Stress und Umweltgifte, insbesondere gegen Schwermetalle produziert wird. Die an der reduzierten Form des GSH beteiligten drei Aminosäuren sind mit speziellen cytoplasmatischen Enzymen verknüpft, die zur Aktivierung ATP benötigen. Ein Anstieg der GSH-Konzentration führt zu einem Anstieg der Glutathion-S-transferase-Aktivität, eines Entgiftungsenzyms. Der GHS-Gehalt wurde nach der Methode von Hissin [vgl. Anal. Biochem. 74, 214-226 (1977)] bestimmt.
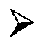 Die *wachstumsstimulierende Wirkung* der Testsubstanzen wurden an menschlichen Fibroblasten untersucht. Dazu wurden in einer ersten Testreihe die Fibroblasten in einem Nährmedium 1 Tag bei 37 °C und 5 Vol.-% CO₂ inkubiert, das Nährmedium gegen ein Medium, welches die Testsubstanzen enthielt, ausgewechselt und wiederum 3 Tage bei 37 °C inkubiert. Anschließend wurde der Proteingehalt in den Zellen sowie die ATP-Konzentration bestimmt.
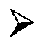 Zur Bestimmung der *überlebensstimulierenden Wirkung* wurden in einer zweiten Testreihe die Fibroblasten zunächst 3 Tage bei 37 °C in einer Nährlösung und dann 3 Tage bei gleicher Temperatur in einer Testlösung inkubiert. Anschließend wurde der Proteingehalt in den Zellen sowie die GSH-Konzentration bestimmt.

Die Ergebnisse sind in Tabelle 2 zusammengefasst. Angegeben sind die Ergebnisse von jeweils 3 Messreihen mit Dreifachbestimmung in %-rel gegenüber einer Blindprobe.

**Tabelle 2**

| **Wachstums- und überlebensstimulierende Wirkung (Angaben in %- rel.)** | | | | | |
|---|---|---|---|---|---|
| **Extrakt** | **Konz. % w/v** | **Testreihe 1** | | **Testreihe 2** | |
| | | Proteine | ATP | Proteine | GSH |
| Blindprobe | 0 | 100 | 100 | 100 | 100 |
| H1 | 0,03 | 112 | 101 | 130 | 123 |
| H2 | 0,01 | 100 | 130 | | |
| H2 | 0,03 | | | 111 | 131 |
| H3 | 0,1 | | | 125 | 177 |
| H4 | 0,1 | 129 | 118 | | |
| H5 | 0,1 | | | 126 | 154 |
| H6 | 0,1 | 153 | 182 | | |
| H7 | 0,1 | | | 120 | 146 |

Die Beispiele zeigen, dass die Extrakte den Metabolismus im Hinblick auf das Wachstum und den Schutz der Fibroblasten stimulieren.

### C. Anti-inflammatorische Wirkung

Im Verlauf einer cutanen Inflammation werden Leucocyten, wie beispielsweise die polymorphonuclearen neutrophilen Granulocyten (PMN) durch Peptide wie etwa Cytokine stimuliert, Botschafterstoffe wie z.B. Leukotrien auszusenden, die von aktivierten oder nekrotischen Zellen in der Dermis freigesetzt werden. Diese aktivierte PMN setzen nicht nur pro-inflammatorische Cytokine, Leukotriene und Proteasen, sondern auch ROS, wie z.B. Superoxide und Hypochloritanionen frei, welche die Aufgabe haben, eingedrungene pathogene Keime oder Pilze zu vernichten. Diese Aktivität der PMN während der Inflammation ist als sogenannter Atmungsausbruch ("respiratory burst") bekannt und kann zu zusätzlichen Schäden im Gewebe führen. Zur Untersuchung, inwiefern die Extrakte den Atmungsausbruch verhindern oder mindern können, wurde eine Zelllinie von menschlichen leukemischen Granulocyten dieser PMN zusammen mit den Testsubstanzen bei 37 °C von 5 Vol.-% CO₂ inkubiert. Nach Auslösung des Atmungsausbruchs durch Zugabe eines Hefeextraktes (Zymosan) zur Zelllösung, wurde die Freisetzung von Superoxidanionen über deren Reaktion mit Luminol bestimmt. Die Ergebnisse sind in Tabelle 3 zusammengefasst. Angegeben sind die Zellzahlen sowie die Menge an freigesetzten ROS in %-rel zum Standard als Mittelwert einer Messreihe mit Dreifachbestimmung.

**Tabelle 3**

| **Anti-inflammatorische Wirkung (Angaben in %-rel.)** | | | |
|---|---|---|---|
| Testprodukt | Konz. **% w/v** | **Zellzahlen** | **Freigesetzte ROS** |
| Blindprobe | 0 | 100 | 100 |
| H1 | 0,1 | 102±4 | 77±17 |
| H3 | 0,1 | 96±6 | 52±18 |
| H4 | 0,1 | 95±9 | 40±37 |
| H5 | 0,1 | 100±7 | 57±14 |

Die Ergebnisse zeigen, dass die Extrakte einen starken inhibierenden Einfluss auf den Atmungsausbruch menschlicher Granulocyten besitzen, ohne die Granulocyten zu schädigen.

### E. Zellschutz vor UVA-Strahlung

Gegenstand der folgenden in-vitro Untersuchungen war festzustellen, ob die Extrakte keimender Pflanzen menschliche Fibroblasten vor oxidativem Stress, speziell der Einwirkung von UVA-Strahlen schützen können. UVA wurde deswegen als Stressfaktor gewählt, da die Strahlen bis in die Dermis eindringen und dort vor allem eine Lipoperoxdation der Cytoplasmamembranen bewirken. Die gebildeten Lipoperoxide zerfallen in Malonaldialdehyde (MDA), die für die Retikulation vieler Biomoleküle, wie z.B. Proteine (Enzyminhibierung) oder Nucleinbasen (Mutagenese) verantwortlich sind. Zur Versuchsdurchführung wurde eine Fibroblastenkultur mit fötalem Kalbsserum angesetzt und 2 Tage später mit den Testsubstanzen geimpft. Nach einer Inkubation von 36 h bei 37 °C und einem CO₂-Level von 5 Vol.-% wurde das Nährmedium durch eine Elektrolytlösung ersetzt und die Fibroblasten mit einer definierten UVA-Strahlungsmenge geschädigt (3-15 J/cm²). Nach dem Ende der Bestrahlung wurde die Menge an gebildetem MDA in der überstehenden Lösung durch Umsetzung mit Thiobarbitursäure und der Gehalt an Proteinen im Zellhomogenisat nach der Bradford-Methode bestimmt. Die Ergebnisse sind in Tabelle 4 zusammengefasst und verstehen sich als %-rel gegenüber dem Standard. Angegeben ist der Mittelwert von zwei Messreihen mit Dreifachbestimmung.

**Tabelle 4**

| **Wirkung gegen UVA-Strahlen (Angaben in %-rel.)** | | | |
|---|---|---|---|
| **Testprodukt** | **Konz. % w/v** | **Freigesetzt es MDA** | **Cellulare Proteine** |
| Kontrolle ohne UVA | | 0 | 100 |
| Kontrolle mit UVA | | 100 | 101 |
| H6 + UVA | 0,03 | 57 | 146 |

Die Ergebnisse zeigen, dass die Extrakte einen nachhaltig positiven Einfluss bei der Bekämpfung von oxidativem Stress besitzen, ohne dabei die Fibroblasten zu schädigen.

### E. Zellschutz vor UVB-Strahlung

UVB-Bestrahlung (280 bis 320 nm) induziert eine cutane Inflammation hauptsächlich durch die Aktivierung der Enzyme Phospholipase A2 oder PLA2, welche aus den Zellwänden Arachidonsäure freisetzen. Diese wird durch die Einwirkung von Cyclooxigenasen in Prostagladine umgewandelt, die ihrerseits von den Zellen abgesondert werden. Die Fixierung von Prostagladinen des PGE2-Typs auf speziellen Hautrezeptoren führt zu Rötungen und Schwellungen der Haut, wie sie auch bei einem Sonnenbrand auftreten. In Zellkulturen ist der Effekt von UVB-Strahlung mit der Freisetzung von cytoplasmatischen Enzymen, insbesondere von Lactat Dehydrogenase (LDH) verbunden. Zur Untersuchung der Anti-UVB-Wirksamkeit der Extrakte menschliche Keratinocyten in einem Nährmedium (DMEM + FCS) über 3 Tage bei 37 °C und 5 Vol.-% CO₂) inkubiert. Anschließend wurde das Nährmedium gegen eine Elektrolytlösung ausgetauscht, welche die Testsubstanz enthielt und die Keratinocyten mit UVB-Strahlung geschädigt (50 mJ/cm²). Nach einer Inkubationszeit von weiteren 24 h wurde die Zellzahl nach Trypsination und die Menge an freigesetztem LDH in der überstehenden Flüssigkeit auf spektrometrischem Wege bestimmt. Die Ergebnisse sind in Tabelle 5 zusammengefasst. Angegeben ist der Mittelwert von zwei Messreihen mit Dreifachbestimmung. Die Angaben verstehen sich als %-rel zu einer Blindprobe.

**Tabelle 5:**

| **Wirkung gegen UVB-Strahlen (Angaben in %-rel.)** | | | |
|---|---|---|---|
| **Extrakt** | **Konz. % w/v** | **Anzahl Keratinocyten** | **Freigesetztes LDH** |
| Blindprobe ohne UVB | 0 | 100 | 0 |
| Blindprobe mit UVB | 0 | 23±5 | 100±0 |
| H1 + UVB | 0,03 | 98±10 | 19±1 |
| H2 + UVB | 0,1 | 39±1 | 52±15 |
| H3 + UVB | 0,1 | 65±2 | 50±1 |
| H5 + UVB | 0,1 | 83±1 | 29±14 |
| H7 + UVB | 0,1 | 104±2 | 24±0 |

Die Ergebnisse zeigen, dass die Extrakte menschliche Keratinocyten ganz erheblich vor dem Einfluss von UVB-Strahlung schützen und daher einen anti-inflammatorischen Effekt besitzen.

### F. Immunstimulation

Unter Immunstimulation sind biochemische Abläufe zu verstehen, bei denen Botenstoffe, wie beispielsweise Betaglucane die körpereigenen Abwehrkräfte stimulieren, um beispielsweise toxische Stoffe zu binden und auszuscheiden und die Erneuerung von Hautzellen zu beschleunigen. Es ist bekannt, dass Organismen diese Fähigkeit mit zunehmendem Alter verloren geht. Die Immunstimulation kann in-vitro an Hand von menschlichen Leukozyten beobachtet werden, welche zuvor mit einem Hefeextrakt (Zymosan) aktiviert worden sind [vgl. Capsoni et al. Int.J.Immunopharm. 10(2), 121-133 (1998)]. Eine Kultur polymorphonuclearer neutrophiler Granulocyten (PMN) wurde zusammen mit den Testsubstanzen über einen Zeitraum von 24 h bei 37 °C und 5 Vol.-% CO₂ inkubiert. Durch Zugabe von Zymosan wurde der Atmungsausbruch ausgelöst. Nach 30 min wurde die PMN-Zahl durch einen automatischen Zellzähler und die Menge an freigesetzten reaktiven Sauerstoffspezies (ROS) in der überstehenden Flüssigkeit mit Hilfe von Luminol spektroskopisch bestimmt. Die Ergebnisse sind in Tabelle 6 zusammengefasst und verstehen sich als %-rel gegenüber dem Standard. Angegeben ist der Mittelwert von zwei Messreihen mit Dreifachbestimmung.

**Tabelle 6**

| **Immunstimulation (Angaben in %-rel.)** | | | |
|---|---|---|---|
| **Testprodukt** | **Konz. % w/v** | **Anzahl Leukozyten** | **Freigesetzte ROS** |
| Blindprobe | 0 | 100 | 100 |
| H1 | 0,01 | 97±3 | 229±39 |
| H3 | 0,001 | 95±5 | 144±29 |
| H5 | 0,001 | 103±4 | 141±44 |
| H7 | 0,001 | 104±4 | 154±34 |

Die Ergebnisse zeigen, dass die Testsubstanzen das Immunsystem stimulieren und die körpereigenen Abwehrkräfte insbesondere der Hautzellen nachhaltig stärken.

### G. Aktivierung der Lipolyse

Unter Lipolyse versteht man die Entfernung von Triglyceriden aus den Adipocyten. Wesentliche Bedeutung kommt dabei der Triglycerid Lipase zu, die die Glyceride in freie Fettsäuren und Glycerin spaltet, die dann über den Blutkreislauf abtransportiert werden. Die Fettsäuren können dann beispielsweise in den Muskelzellen verbrannt werden und dienen somit als Energiespeicher. Zur Untersuchung der lipolytischen Aktivität wurden Adipocyten aus menschlichem subkutanem Gewebe isoliert [vgl. Rodbell, J.Biol.Chem. 239(2), 375-380 (1964)]. Die entsprechenden Kulturen wurden mit den Testsubstanzen geimpft und über einen Zeitraum von 90 min bei 37 °C inkubiert. Die Menge an freigesetztem Glycerin in der überstehenden Flüssigkeit wurde spektroskopisch bestimmt [vgl. Carpéné et al., J.Pharmacol. (Paris), 12(2), 219-224 (1981)]. Die Ergebnisse sind in Tabelle 7 zusammengefasst und verstehen sich als %-rel gegenüber dem Standard. Angegeben ist der Mittelwert von zwei Messreihen mit Dreifachbestimmung.

**Tabelle 7**

| **Lipolyse (Angaben in %-rel.)** | | |
|---|---|---|
| **Testprodukt** | **Konz. % w/v** | **Freigesetztes Glycerin** |
| Blindprobe | 0 | 0 |
| H2 | 0,1 | 15 |
| H2 | 0,3 | 50 |
| H3 | 0,1 | 109 |
| H3 | 0,3 | 353 |
| H4 | 0,1 | 36 |
| H4 | 0,3 | 147 |

Die Ergebnisse zeigen, dass die Testsubstanzen die Lipolyse anregen und damit zu einer schnelleren Verfügbarkeit von Zellbrennstoff beitragen.

In Tabelle 8 sind eine Reihe von Formulierungsbeispiele enthalten.

**Tabelle 8**

| **Beispiele für kosmetische Zubereitungen (Wasser, Konservierungsmittel ad 100 Gew.-%)** | | | | | |
|---|---|---|---|---|---|
| **Zusammensetzung (INCI)** | **A** | **B** | **C** | **D** | **E** |
| **Emulgade® SE** Glyceryl Sterate (and) Ceteareth 12/20 (and) Cetearyl Alcohol (and) Cetyl Palmitate | 5,0 | 5,0 | 4,0 | - | - |
| **Eumulgin® B1** Ceteareth-12 | - | - | 1,0 | - | - |
| **Lameform® TGI** Polyglyceryl-3 Isostearate | - | - | - | 4,0 | - |
| **Dehymuls® PGPH** Polyglyceryl-2 Dipolyhydroxystearate | - | - | - | - | 4,0 |
| **Monomuls® 90-O 18** Glyceryl Oleate | - | - | - | 2,0 | - |
| **Cetiol® HE** PEG-7 Glyceryl Cocoate | - | - | - | - | 2,0 |
| **Cetiol® OE** Dicaprylyl Ether | - | - | - | 5,0 | 6,0 |
| **Cetiol® PGL** Hexyldecanol (and) Hexyldecyl Laurate | - | - | 3,0 | 10,0 | 9,0 |
| **Cetiol® SN** Cetearyl Isononanoate | 3,0 | 3,0 | - | - | - |
| **Cetiol® V** Decyl Oleate | 3,0 | 3,0 | - | - | - |
| **Myritol® 318** Coco Caprylate Caprate | - | - | 3,0 | 5,0 | 5,0 |
| **Bees Wax** | - | - | - | 7,0 | 5,0 |
| **Nutrilan® Elastin E20** Hydrolyzed Elastin | 2,0 | - | - | - | - |
| **Nutrilan® I-50** Hydrolyzed Collagen | - | 2,0 | - | - | - |
| **Gluadin® AGP** Hydrolyzed Wheat Gluten | - | - | 0,5 | - | - |
| **Gluadin® WK** Sodium Cocoyl Hydrolyzed Wheat Protein | - | - | - | 0,5 | 0,5 |
| **Extrakt H1** | 1,0 | 1,0 | 1,0 | - | - |
| **Extrakt H5** | - | - | - | 1,0 | 1,0 |
| **Hydagen® CMF** Chitosan | 1,0 | 1,0 | 1,0 | 1,0 | 1,0 |
| **Magnesium Sulfate Hepta Hydrate** | - | - | - | 1,0 | 1,0 |
| **Glycerin** (86 Gew.-%ig) | 3,0 | 3,0 | 5,0 | 5,0 | 3,0 |
| (A) Softcreme, (B,C) Feuchtigkeitsemulsion, (D,E) Nachtcreme | | | | | |

## Patentansprüche

1. Kosmetische Zubereitungen, enthaltend eine wirksame Menge eines Extraktes von keimenden Pflanzen.

2. Zubereitungen nach Anspruch 1, **dadurch gekennzeichnet, dass** die Pflanzen ausgewählt sind aus der Gruppe, die gebildet wird von Alfalfa, Bambaranuss, Bockshorn, Borretsch, Broccoli, Buchweizen, Chinakohl, Erbse, Erdnuss, Flachs, Fenchel, Gerste, Getreide, Gewürznelke, Hafer, Karotte, Kresse, Linsen, Mais, Melone, Petersilie, Raps, Rettich, Reis, Roggen, Rotkohl, Sellerie, Senf, Sesam, Soja, Sonnenblume, Weizen, und Zwiebel.

3. Zubereitungen nach den Ansprüchen 1 und/oder 2, **dadurch gekennzeichnet, dass** sie die Extrakte in Mengen von 0,01 bis 25 Gew.-% enthalten.

4. Verfahren zur Herstellung von Pflanzenextrakten, bei dem man die keimenden Samen mit Wasser und/oder Alkohol extrahiert, den so erhaltenen Extrakt thermisch behandelt, gegebenenfalls anschließend mit exogenen Enzymen versetzt und gegebenenfalls trocknet.

5. Verwendung von Extrakten keimender Pflanzen zur Herstellung von kosmetischen Zubereitungen.

6. Verwendung von Extrakten keimender Pflanzen zur Stimulation des Zellwachstums und des Zellmetabolismus.

7. Verwendung von Extrakten keimender Pflanzen zur Stimulation der Erneuerung von dermalen Makromolekülen durch die Fibroblasten.

8. Verwendung von Extrakten keimender Pflanzen zur Stimulation der Zellproteinsynthese zum Schutz gegen spontane Alterungseffekte.

9. Verwendung von Extrakten keimender Pflanzen zur Steigerung der Protein- und GSH-Konzentration in den Zellen.

10. Verwendung von Extrakten keimender Pflanzen zur Stimulierung der G6PDH-Aktivität.

11. Verwendung von Extrakten keimender Pflanzen zur Immunmodulation.

12. Verwendung von Extrakten keimender Pflanzen als anti-inflammatorische Wirkstoffe.

13. Verwendung von Extrakten keimender Pflanzen als Wirkstoffe gegen Akne und Rosacea.

14. Verwendung von Extrakten keimender Pflanzen als Antioxidantien.

15. Verwendung von Extrakten keimender Pflanzen zum Schutz von Haut und Haaren gegen den Einfluss von UVA- und UVB-Strahlen.

16. Verwendung von Extrakten keimender Pflanzen zum Schutz von empfindlicher Haut.

17. Verwendung von Extrakten keimender Pflanzen als Anti-Stress-Wirkstoffe.

18. Verwendung von Extrakten keimender Pflanzen zur Stimulation der Synthese und Freisetzung von Hitzeschock-Proteinen.

19. Verwendung von Extrakten keimender Pflanzen als lypolytische Wirkstoffe.

20. Verwendung von Extrakten keimender Pflanzen als Wirkstoffe zur Entschlackung von Körperzellen.

21. Verwendung von Extrakten keimender Pflanzen als Wirkstoffe zur Inhibierung der Melaninsynthese in Haut- und Haarzellen.

22. Verwendung von Extrakten keimender Pflanzen als Wirkstoffe mit östrogenartiger Aktivität.
